Europäisches Patentamt

European Patent Office

Office européen des brevets

(11) Numéro de publication : **0 328 443 B1**

(12)

# FASCICULE DE BREVET EUROPEEN

(45) Date de publication du fascicule du brevet :
**07.01.93 Bulletin 93/01**

(51) Int. Cl.⁵ : **G01B 11/24,** G05B 19/42

(21) Numéro de dépôt : **89400325.0**

(22) Date de dépôt : **06.02.89**

(54) **Procédé de numérisation de la surface d'un objet tridimensionnel et appareil de relevé en vue de sa mise en oeuvre.**

(30) Priorité : **09.02.88 FR 8801897**

(43) Date de publication de la demande :
**16.08.89 Bulletin 89/33**

(45) Mention de la délivrance du brevet :
**07.01.93 Bulletin 93/01**

(84) Etats contractants désignés :
**AT BE DE ES FR GB GR IT NL SE**

(56) Documents cités :
**EP-A- 0 163 076
EP-A- 0 222 498
WO-A-87/01194
FR-A- 2 375 578
FR-A- 2 470 415
FR-A- 2 519 138
LU-A- 78 624
US-A- 3 399 592**

(73) Titulaire : **SA KREON INDUSTRIE
Route des Usines
F-65300 Lannemezan (FR)**

(72) Inventeur : **Brunet, Michel
34 rue de la Bourdette
F-31130 Balma (FR)**
Inventeur : **Cosnard, Eric
Chemin de Limes Lapeyrouse Fossat
F-31180 Castelmaurou (FR)**

(74) Mandataire : **Rodhain, Claude et al
Cabinet Claude Rodhain 30, rue la Boétie
F-75008 Paris (FR)**

## Description

L'invention concerne un procédé de numérisation de la surface d'un objet (ou sujet) tridimensionnel permettant d'engendrer des données numériques représentatives des coordonnées des points de cette surface dans un référentiel tridimensionnel. Elle vise un procédé d'acquisition du relief de l'objet à partir d'un éclairage structuré à faisceaux lamellaires, en particulier lasers, et d'un dispositif de saisie vidéo. Elle s'étend à un appareil de relevé adapté pour délivrer un signal vidéo représentatif de la surface de l'objet, ce signal pouvant être, soit numérisé en temps réel localement ou après télétransmission pour fournir le signal numérique précité, soit enregistré et numérisé ultérieurement au moment de son exploitation. L'invention vise tout particulièrement, mais non exclusivement, à réaliser le relevé d'une tête ou d'un buste humain notamment en vue d'une reproduction par usinage.

Depuis plusieurs années s'est développé un procédé de numérisation d'objets tridimensionnels qui consiste à former sur l'objet une trace lumineuse mettant en évidence une section de celui-ci, à filmer ledit objet au moyen d'un dispositif vidéo de façon à enregistrer l'image de cette trace et à balayer l'ensemble de la surface afin de former successivement des traces lumineuses sur des sections réparties sur la surface et d'en acquérir les coordonnées par leur observation vidéo. On pourra notamment se reporter au EP-A-0 163 076 qui, entre autres, concerne cette technique.

L'avantage d'un tel procédé par rapport à des procédés plus anciens tels que photogrammétrie, technique de moiré... réside essentiellement dans la simplicité et la rapidité d'extraction des coordonnées des points de la surface de l'objet à partir du signal vidéo issu du dispositif vidéo ; par exemple dans les procédés plus anciens ci-dessus évoqués, la restitution des coordonnées fait appel à des techniques lourdes et complexes, nécessitant généralement la présence d'un opérateur humain. Au contraire dans le procédé concerné par l'invention, chaque pixel activé du signal vidéo est directement représentatif des coordonnées d'un point de l'objet dans la trace lumineuse (coordonnées bidimensionnelles) et le traitement se cantonne à une codification numérique de ces pixels activés et à leur mémorisation associée à des informations relatives au mouvement de balayage de la surface (qui sont représentatives de la troisième coordonnée).

Toutefois, dans le cas de certains objets convexes ou d'objets ayant des reliefs accentués, les systèmes existants de mise en oeuvre du procédé sus-visé ne permettent pas d'acquérir de façon complète tous les points de l'objet et ne fournissent que des résultats partiels. Par exemple dans l'application à la numérisation d'une tête ou d'un buste, ces systèmes ne permettent de voir que le visage et laissent non apparents le dessous du menton et le crâne ; de plus, certaines parties du visage (ailes du nez, partie postérieure du maxillaire, oreilles) se trouvent masquées dans la plupart de ces systèmes antérieurs. Pour pallier cet inconvénient ou peut prévoir plusieurs faisceaux lasers ou plusieurs caméras vidéo en vue d'enrichir les données issues du système. Tel est le cas du EP-A-0 163 076 précité. Toutefois, la technique qui y est décrite implique, pour le calcul, de connaître tous les paramètres géométriques (angles, etc.) de l'ensemble laser/caméra, avec toutes les imprécisions associées.

L'invention vise un procédé perfectionné de numérisation permettant de s'affranchir totalement de la géométrie matérielle du système et d'éviter la détermination des coordonnées recherchées par des calculs trigonométriques.

Elle s'étend à un appareil pour la mise en oeuvre de ce procédé.

Le procédé de numérisation visé par l'invention a pour objectif d'engendrer un ensemble de données numériques représentatives des coordonnées des points de la surface d'un objet (ou sujet) tridimensionnel dans un référentiel tridimensionnel. Ce procédé est du type énoncé dans le préambule de la revendication 1, qui correspond aux enseignements du EP-A-0 163 076 précité

Le procédé conforme à la présente invention est caractérisé par les étapes spécifiques de calibration énoncées dans le préambule de la revendication 1

Comme on le comprendra mieux plus loin, ces caractéristiques combinées résolvent de manière satisfaisante le problème consistant à s'affranchir totalement de la géométrie physique du système, grâce à la procédure originale dite de calibration qui permet d'obtenir des informations représentatives de la géométrie du système sans avoir à en mesurer les divers paramètres.

Ainsi, les mesures de paramètre se réduisent à la simple définition (une fois pour toute) d'une mire de calibration, et à sa numérisation par le procédé de l'invention ; aucune mesure physique de paramètres géométriques (distance, angle) n'est plus à effectuer sur le système, ce qui représente en pratique un avantage considérable.

D'autres caractéristiques et fermes de réalisation avantageuses sont énoncées dans les sous-revendications 2 à 15.

L'invention s'étend à un appareil de relevé tridimentionnel de la surface d'un objet, conformément à la revendication 16.

D'autres caractéristiques, buts et avantages de l'invention ressortiront de la description qui suit en référence aux dessins annexés, lesquels illustrent, à titre d'exemples non limitatifs, des modes de mise en oeuvre du procédé de l'invention et des modes de réalisation de l'appareil ; sur ces dessins qui font partie intégrante de la présente description :

- la figure 1 est une vue en perspective d'un appareil de relevé conforme à l'invention,
- la figure 2 est une vue en perspective à échelle plus grande d'une partie de l'appareil,
- la figure 3 est une vue de côté de l'appareil,
- la figure 4 en est une vue postérieure avec arraché partiel du piétement,
- la figure 5 en est une vue de dessous,
- la figure 6 est une vue de détail en coupe d'un dispositif laser,
- les figures 7 et 8 sont des vues schématiques illustrant l'agencement géométrique des faisceaux d'éclairage et angles de prises de vue vidéo,
- la figure 9 est un synoptique de la chaîne de traitement du signal vidéo,
- la figure 10 est une vue schématique illustrant le processus de partition,
- la figure 11 est une vue schématique visant un autre mode de réalisation et illustrant l'agencement géométrique des moyens d'éclairage et dispositif vidéo,
- la figure 12 est un synoptique de la chaîne de traitement dans le cas de l'appareil de la figure 11,
- les figures 13, 14, 15a, 15b, et 16 illustrent la procédure de calibration et les moyens adaptés à sa mise en oeuvre.

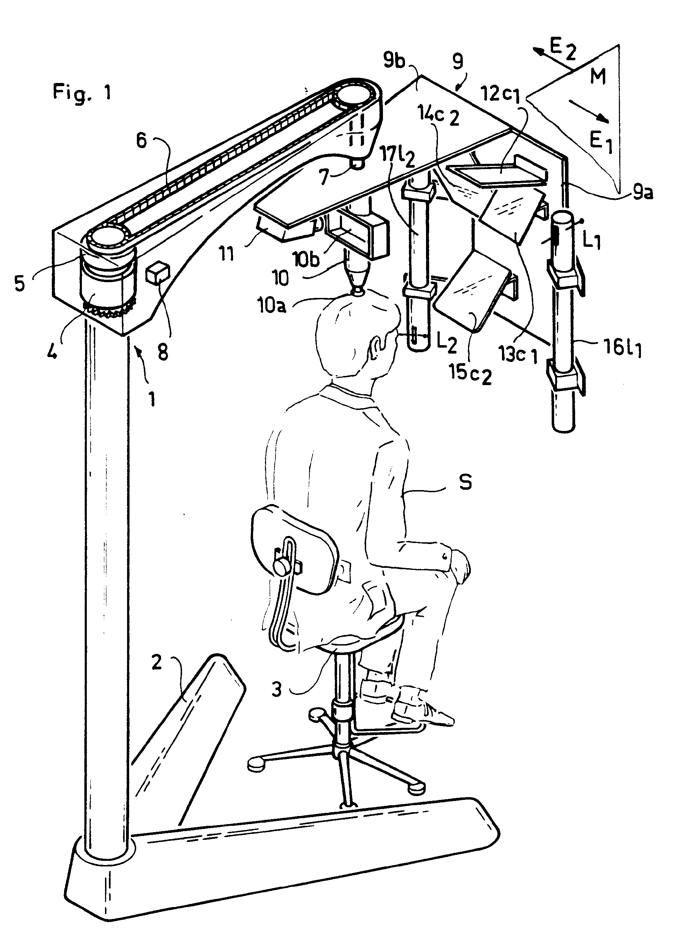

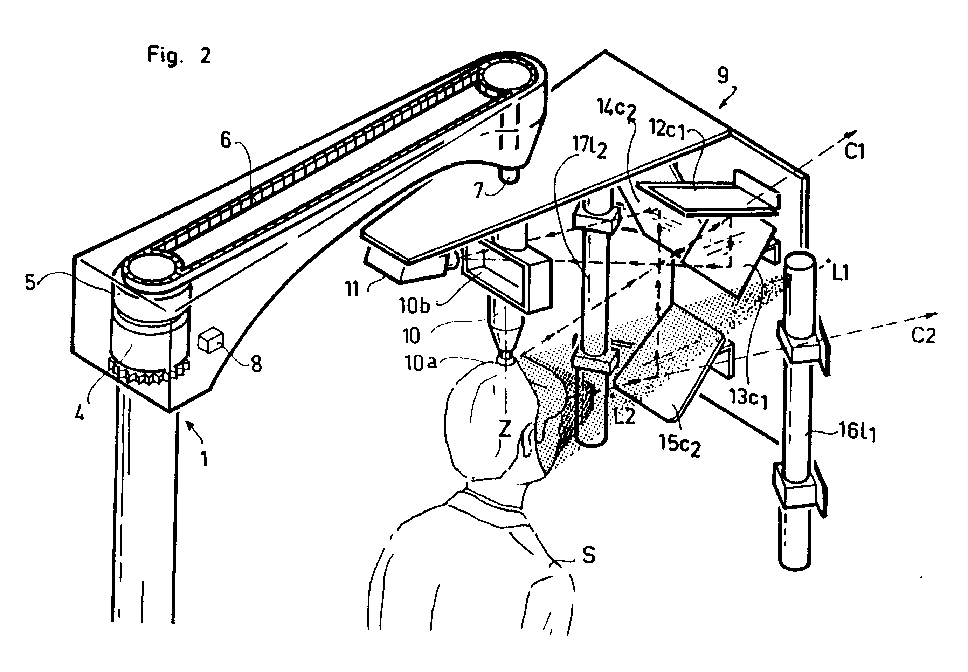

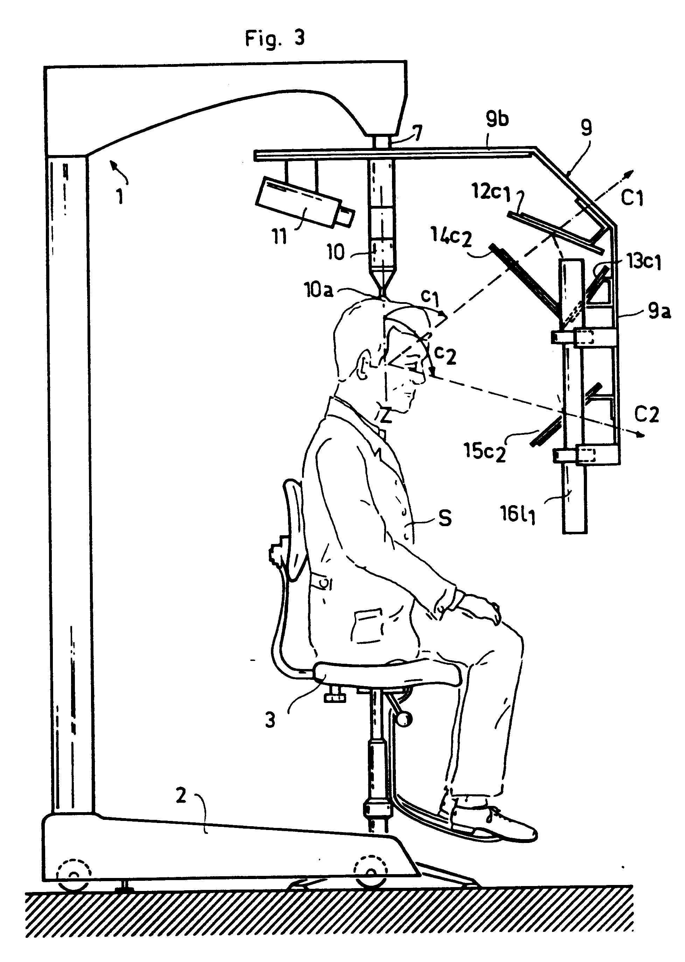

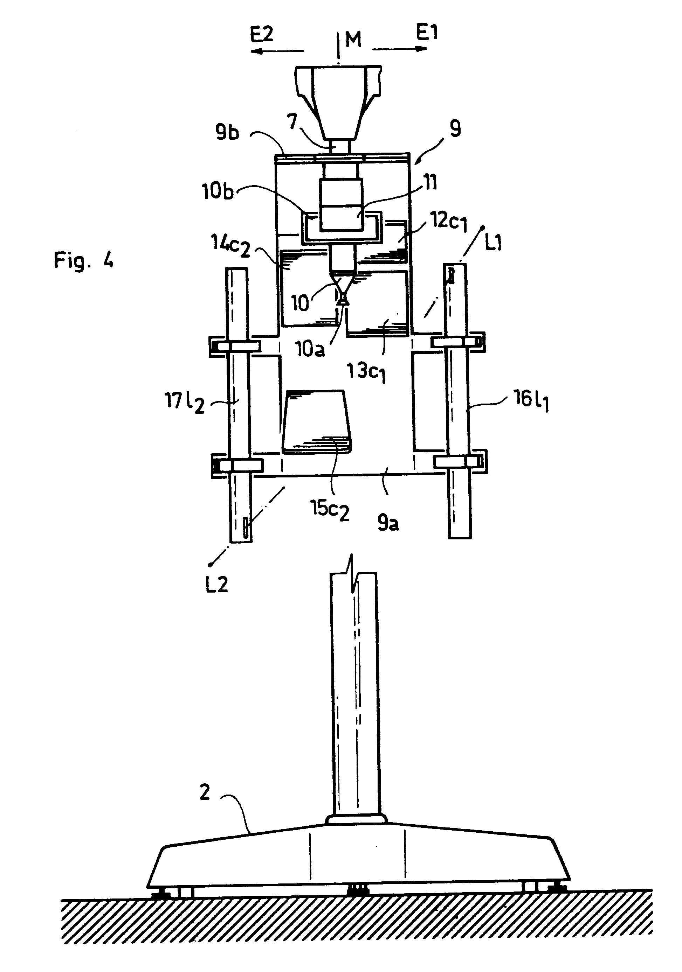

L'appareil de relevé représenté à titre d'exemple aux figures 1 à 6 est destiné au relevé d'une tête humaine, notamment en vue de sa reproduction par machine-outil à commande numérique ; cet appareil comprend une potence 1 maintenue par un piétement 2 en vue de surplomber un sujet S assis sur un siège 3. A sa partie arrière, la potence 1 constituée par un carter fermé contient un moteur électrique 4 associé à un réducteur 5. Ce réducteur est accouplé à un système de transmission 6, en l'exemple à poulies et courroie crantée, en vue d'entraîner un arbre de sortie vertical 7 qui définit un axe de rotation Z contenu dans le plan médian M et passant par la verticale du siège 3.

Dans l'exemple décrit, un capteur de position angulaire 8 est couplé à l'arbre du moteur électrique en vue de délivrer à chaque instant un signal représentatif de la position angulaire de l'arbre de moteur par rapport à un repère fixe et donc représentatif de la position angulaire $\theta$ de l'arbre de sortie 7 par rapport au sujet S. Ce capteur est en particulier constitué par un générateur d'impulsions électriques de fréquence proportionnelle à la vitesse de rotation (roue phonique montée sur l'arbre du moteur et capteur inductif). Il est à noter que ce capteur peut le cas échéant être supprimé dans le cas d'une vitesse de rotation constante, la position angulaire $\theta$ étant alors déduite du temps écoulé, directement mesuré par la fréquence du défilement des images vidéo.

L'arbre vertical 7 porte un support tournant 9 possédant une extension frontale verticale 9a et une extension supérieure horizontale 9b. Un organe de centrage 10 est fixé sur l'extension supérieure 9b en position verticale, coaxiale à l'arbre 7 ; cet organe est destiné à venir en appui au-dessus de la tête du sujet, grâce à une pastille de caoutchouc 10a montée libre en rotation. Le sujet peut ainsi se centrer en prenant appui contre cette pastille afin de demeurer parfaitement immobile pendant le relevé. En l'exemple, l'organe 10 est constitué par un tube, sur la longueur duquel est intercalé un profil ajouré 10b en vue de dégager le chemin optique entre une caméra 11 fixée sous l'extension supérieure 9b et un jeu de miroirs fixés sur l'extension frontale 9a.

La caméra 11 est ainsi fixée à l'opposé des miroirs par rapport au sujet, dans le plan médian M de l'ensemble tournant porté par le support 9. (La caméra 11 est inversée de façon à produire une image à l'endroit si l'on y raccorde un moniteur de contrôle).

L'extension frontale 9a porte deux jeux de deux miroirs :
- miroirs $12c_1$ et $13c_1$ engendrant un premier point de vue virtuel $C_1$,
- miroirs $14c_2$ et $15c_2$ engendrant un second point de vue $C_2$.

Les miroirs $12c_1$ et $13c_1$ sont disposés d'un côté du plan médian M avec un bord affleurant celui-ci (en l'exemple côté droit vu de la caméra), de façon que le champ de prise de vue réfléchi par ces miroirs corresponde au demi-espace $E_1$ situé du côté droit du plan médian M.

Les miroirs $14c_2$ et $15c_2$ sont disposés de l'autre côté (gauche) de façon que le champ de prise de vue défini par ces miroirs corresponde au demi-espace $E_2$ situé du côté gauche du plan médian M. Ainsi, les champs attachés aux deux points de vue $C_1$ et $C_2$ sont disjoints et correspondent à deux demi-images contiguës dans la caméra 11.

De plus, les miroirs $12c_1$ et $13c_1$ sont géométriquement disposés de façon à engendrer un point de vue $C_1$ en plongée par rapport au sujet ; à cet effet, l'angle de prise de vue (angle $c_1$ formé par l'axe optique et par l'axe de rotation Z) est inférieur à 90° et en particulier de l'ordre de 65°. Au contraire, les miroirs $14c_2$ et $15c_2$ sont géométriquement disposés de façon à engendrer un point de vue $C_2$ en contreplongée, l'angle de prise de vue $c_2$ étant supérieur à 90° et en particulier de l'ordre de 115°.

De plus, ces miroirs sont agencés de façon que les longueurs des chemins optiques, gauche et droit, soient voisines pour autoriser une mise au point unique.

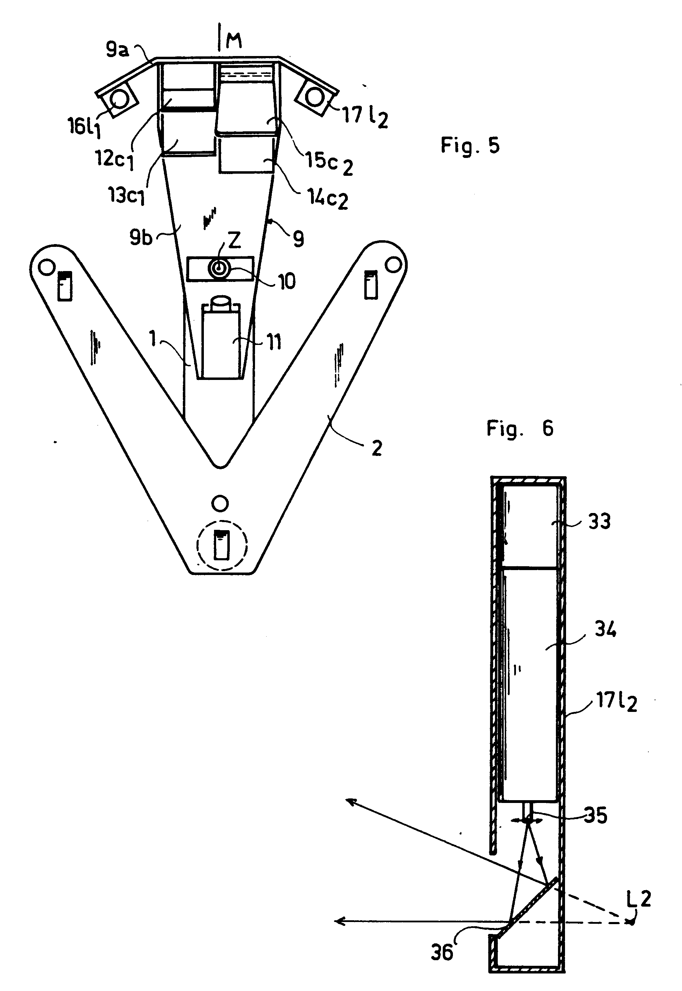

Par ailleurs, l'extension 9a porte de part et d'autre du plan médian M deux dispositifs lasers hélium/néon à lentilles cylindriques $161_1$ et $171_2$, disposés tête-bêche ; ces dispositifs lasers sont connus en eux-mêmes et fournissent chacun un faisceau laser lamellaire. Comme l'illustre la figure 6 qui représente en coupe un exemple de dispositif laser à faisceau lamellaire (alimentation 33, tube laser 34, lentille cylindrique 35, miroir 36), on a désigné par $L_1$ et $L_2$ les sources lasers fictives obtenues en supposant chaque faisceau directement issu d'un point ; en pratique, la source laser réelle de chaque dispositif se trouve décalée par la présence d'un système optique 35, 36 que contiennent ces dispositifs lasers à faisceau lamellaire ; toutefois, dans la géométrie caractéristique de l'invention, ce sont les positions de ces sources fictives $L_1$ et $L_2$ qui sont à considérer (de même que pour les points de vue fictifs $C_1$ et $C_2$ dans le cas du dispositif vidéo).

La caméra 11 est équipée d'un filtre interférentiel accordé à la longueur d'onde des lasers, de façon à la rendre spécifiquement sensiblement à cette longueur d'onde et à s'affranchir des problèmes d'éclairage ambiant.

Le dispositif laser $161_1$ situé à droite du côté du point de vue $C_1$ (en plongée) éclaire le sujet en plongée sous un angle $l_1$ (par rapport à l'axe de rotation Z) sensiblement égal à l'angle de prise de vue $c_1$. L'autre dispositif situé à gauche du côté du point de vue $C_2$ (en contreplongée) éclaire le sujet en contreplongée sous un angle $l_2$ sensiblement égal à l'angle de prise de vue $c_2$.

Les deux dispositifs $161_1$ et $171_2$ sont disposés, tête-bêche, symétriquement par rapport au plan médian M de façon que les faisceaux lumineux correspondants soient contenus dans des plans verticaux $P_1$ et $P_2$ passant par l'axe de rotation Z et formant des angles de parallaxes $p_1$, $p_2$, en l'exemple sensiblement égaux à 30° de part et d'autre du plan M. Les faisceaux forment ainsi deux traces lumineuses sur le sujet, situées de part et d'autre du plan médian M.

Il est à noter qu'un joint tournant classique est inclus dans l'arbre de sortie 7 en vue de permettre l'alimentation électrique de la caméra 11 et des dispositifs laser $161_1$ et $171_2$ et le transfert du signal vidéo issu de la caméra.

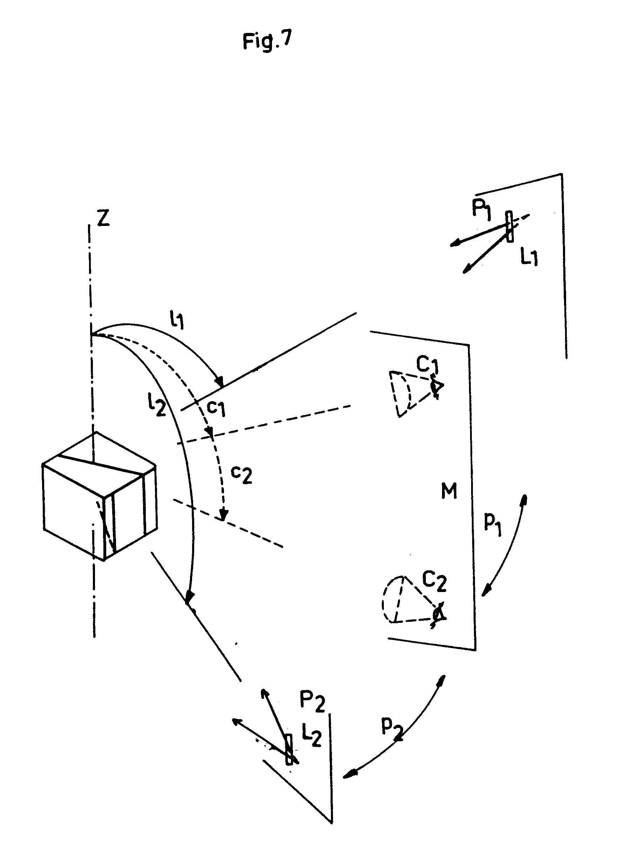

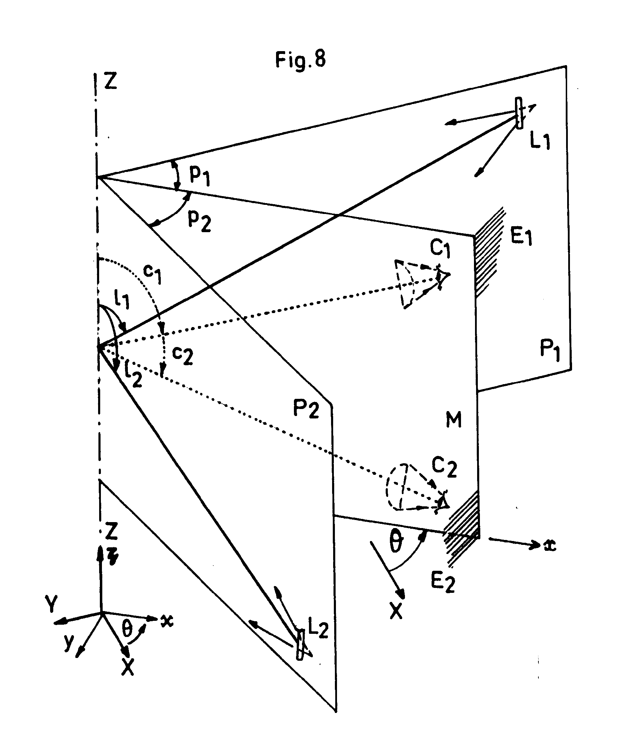

L'agencement géométrique des points de vue $C_1$, $C_2$ et sources $L_1$ et $L_2$ est symbolisé aux figures 7 et 8 afin de le rendre plus clair. Le repère XYZ est un repère fixe lié au sujet et le repère xyz un repère mobile lié au support tournant 9, ces repères étant supposés confondus à l'instant initial et formant un angle $\theta$, à un instant t de la rotation correspondant à une prise d'image donnée.

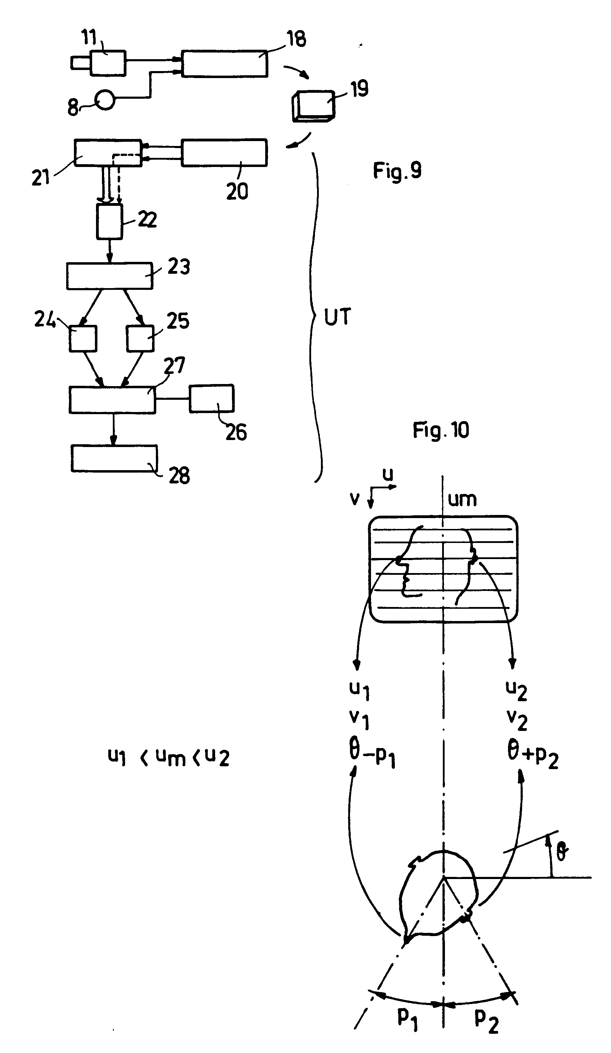

La figure 9 illustre un exemple de chaîne de traitement du signal vidéo issu de la caméra 11, et des informations de position angulaire provenant du capteur 8. Dans cet exemple, le signal vidéo est enregistré au moyen d'un magnétoscope 18 sur une bande magnétique traditionnelle (symbolisée en 19), le signal de position angulaire constitué par une fréquence audio proportionnelle à la vitesse étant enregistré sur la piste SON de cette bande magnétique 19.

Cet enregistrement effectué directement en aval de la caméra 11 et du capteur 8 permet de supprimer tout traitement au niveau de l'appareil de relevé et donc de réduire le prix de cet appareil qui est mis à la disposition d'un réseau commercial en contact avec le public. Les bandes 19 sont centralisées dans un ou des centres spécialisés équipés, d'une part, d'une unité de traitement UT telle que décrite ci-après, d'autre part, de machines-outils à commande numérique, aptes à exploiter la base de données numériques obtenue pour réaliser les reproductions en relief.

Dans le centre spécialisé, chaque bande 19 est lue sur en magnétoscope 20 qui délivre les signaux vers un convertisseur analogique/numérique 21. Pour chaque image, ce convertisseur code à chaque ligne ($v_1$, $v_2$...) les positions des pixels activés ($u_1$, $u_2$...) et les écrit dans une mémoire de stockage 22. Ce codage peut être effectué en écrivant séquentiellement dans les cellules mémoires, le code de la position de chaque pixel sur sa ligne ($u_1$, $u_2$...), l'adresse de la cellule étant représentative du numéro de ligne ($v_1$, $v_2$...).

Le convertisseur 21 comprend un compteur en vue de numériser le signal de position de la piste SON, ce signal numérisé (représentatif de $\theta$) étant stocké en en-tête de chaque image.

Il est à noter que, dans le cas d'une vitesse uniforme de rotation, le système peut fonctionner en l'absence de signal de position ; le compteur précité comptabilise alors les "top de synchronisation trame" afin d'identifier chaque image par sa position temporelle.

Les positions numérisées ($u_1$, $v_1$ ; $u_2$, $v_2$...) stockées, pour chaque image, dans la mémoire 22 sont délivrées vers un séparateur de traces 23 qui réalise une partition de celles-ci en deux fichiers-tampons 24 et 25, chacun contenant les positions numérisées des traces formées par un laser ($L_1$ ou $L_2$) et les positions angulaires $\theta$ correspondantes. Cette partition, illustrée à la figure 10, est effectuée en comparant pour chaque ligne ($v_1$, $v_2$...) les valeurs des positions numérisées ($u_1$, $u_2$...) à la valeur médiane ($u_m$) correspondant à la position de l'axe de rotation Z dans l'image. En cas de valeur inférieure, les positions numérisées et l'en-tête $\theta$ sont stockées dans le fichier-tampon 24 ; en cas de valeur supérieure, elles sont stockées dans le fichier-tampon 25.

Par ailleurs, des informations représentatives de la géométrie de l'ensemble dispositif vidéo/faisceaux lasers sont stockées dans une mémoire 26 et délivrées vers un micro-ordinateur 27 ; en l'exemple, ces informations sont représentatives des parallaxes des faisceaux $p_1$, $p_2$, et des angles de plongée et de contreplongée $c_1$ et $c_2$ des deux points de vue et sont définies par des mesures préalables d'angles et de distance.

Pour chaque image, ces informations de géométrie associées à l'information de position angulaire $\theta$ permettent au micro-ordinateur 27 de calculer (par des calculs trigonométriques classiques) des informations, dites spécifiques, attachées à chaque faisceau laser et au point de vue correspondant pour l'image considérée. Ces informations caractérisent la trace qui est formée par ledit faisceau et qui est observée dudit point de vue ; elles sont associées aux positions numérisées correspondant à cette trace en vue de fournir la troisième coordonnée. Ces coordonnées sont stockées dans une banque de données tridimensionnelles 28.

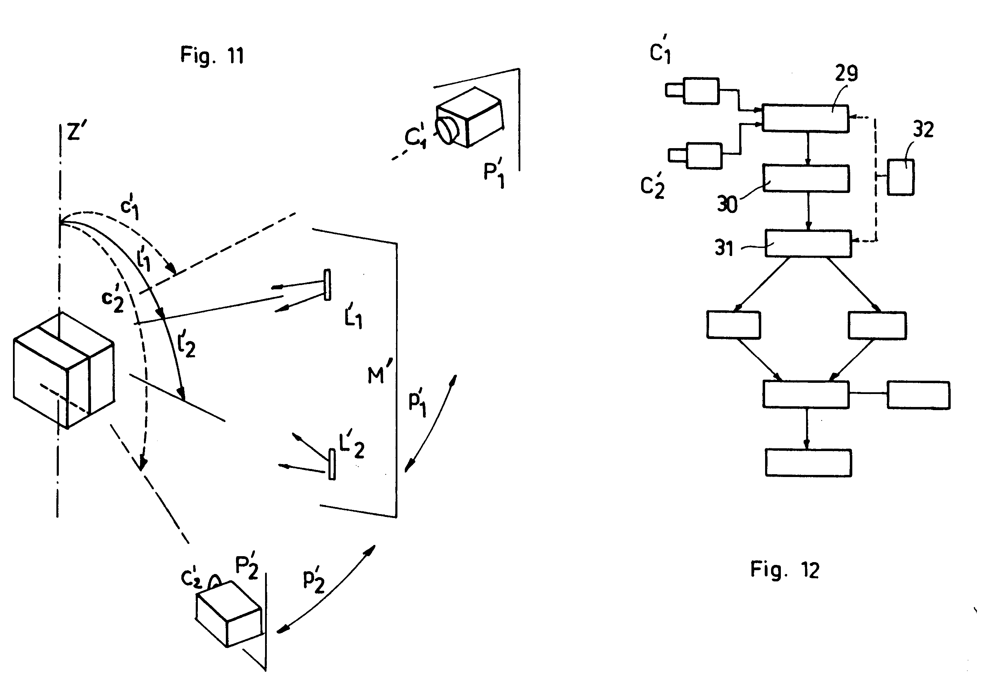

La figure 11 illustre un autre mode de réalisation de l'appareil. La structure mécanique générale de l'appareil est similaire, mais l'on éclaire la surface de l'objet (ou du sujet) par deux faisceaux lasers qui, dans ce cas, sont situés dans un plan médian M' contenant l'axe de rotation Z' (ou situés au voisinage d'un tel plan) à partir de deux sources lasers L'$_1$, L'$_2$ situées dans ce plan médian et décalées en hauteur, l'une en plongée (angle de plongée : l'$_1$), l'autre en contreplongée (l'$_2$).

L'on filme alors l'objet au moyen de deux caméras positionnées selon deux points de vue C'$_1$, C'$_2$ situés de part et d'autre du plan médian M' à des hauteurs différentes pour donner un angle de prise de vue en plongée c'$_1$ et en contreplongée c'$_2$. Ces angles ont des valeurs similaires à celles déjà fournies pour l'autre mode de réalisation ; il en est de même pour les angles de parallaxe p'$_1$, p'$_2$.

Dans ce cas, l'objet est filmé des deux points de vue C'$_1$, C'$_2$ de façon que les champs de prises de vue se recouvrent au niveau de l'objet et contiennent la trace des deux faisceaux lumineux.

La figure 12 illustre le cas d'un traitement en ligne en l'absence de capteur de position (vitesse de rotation uniforme). Les signaux vidéo issus des caméras C'$_1$, C'$_2$ sont multiplexés dans un multiplexeur 29 en amont d'un convertisseur analogique/numérique 30 et, pour chaque image, la partition des positions numérisées est réalisée en fonction de la caméra dont est issue l'image considérée, grâce à un aiguillage 31 ; un séquenceur 32 synchronise le multiplexage d'entrée et l'aiguillage de sortie du convertisseur 30 en fonction des top de synchronisation de trame. La suite du traitement est similaire au cas précédent.

Dans les modes de réalisation décrits précédemment, les calculs effectués par le micro-ordinateur 27 sont des calculs classiques de type trigonométrique et les informations nécessaires représentatives de la géométrie de l'appareil sont des distances et des angles ($p_1$, $p_2$, $c_1$, $c_2$) préalablement mesurés et stockés dans la mémoire 26.

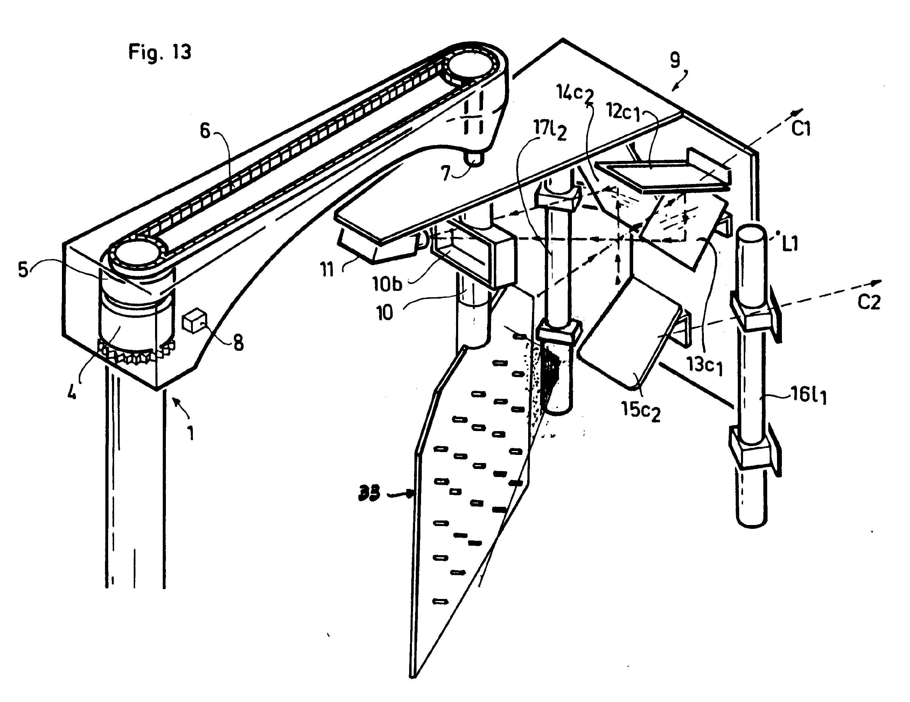

Les figures 13, 14, 15a, 15b et 16 ont trait à un autre mode de réalisation, présentant un intérêt industriel essentiel, car il évite les mesures des paramètres géométriques sus-évoqués et écarte les erreurs afférentes. De plus, il permet de prendre en compte des optiques de caméra avec anamorphose, qui augmentent la résolution horizontale $\frac{\partial u}{\partial x}$ et donc la précision.

Dans ce mode de réalisation, dans une phase préalable (qui peut être renouvelée de temps en temps), on substitue à l'objet à numériser une mire de calibration 33 présentant des reliefs de géométrie et de dimensions connues préalablement mémorisées. Par exemple, on peut utiliser comme mire une plaque 33a portant sur une face un maillage de piges 33b, chacune constituée par un téton cylindrique.

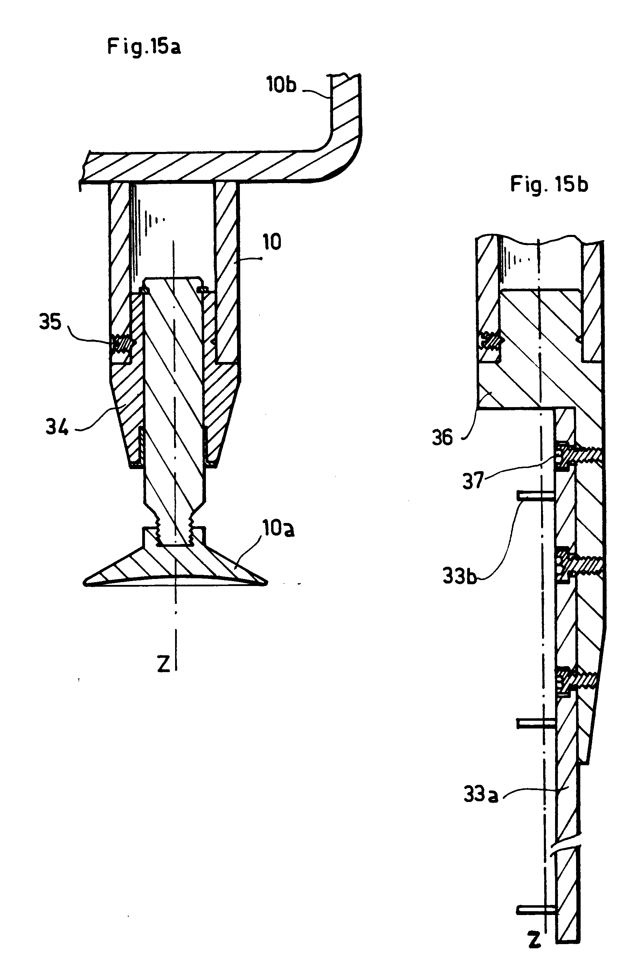

La mire 33 est disposée à l'extrémité du tube 10 déjà décrit, à la place de la ventouse. A cet effet, comme le représente la figure 15a, la ventouse 10a est fixée sur un embout 34 qui est amovible grâce à une vis-pression 35.

Un autre embout 36 tel que représenté à la figure 15b peut être disposé à l'extrémité du tube 10 pour réaliser l'opération préalable de calibration. Cet embout porte la plaque de mire 33a qui est fixée par des goujons 37 de façon à être en retrait par rapport à l'axe vertical Z, les piges 33b étant de longueur suffisante pour intercepter le faisceau laser considéré passant par l'axe Z. Il est à noter que le tube 10 peut permettre de fixer d'autres types d'embouts de centrage en vue de numériser d'autres objets que des têtes, par exemple objet inerte fixé par un embout spécifique.

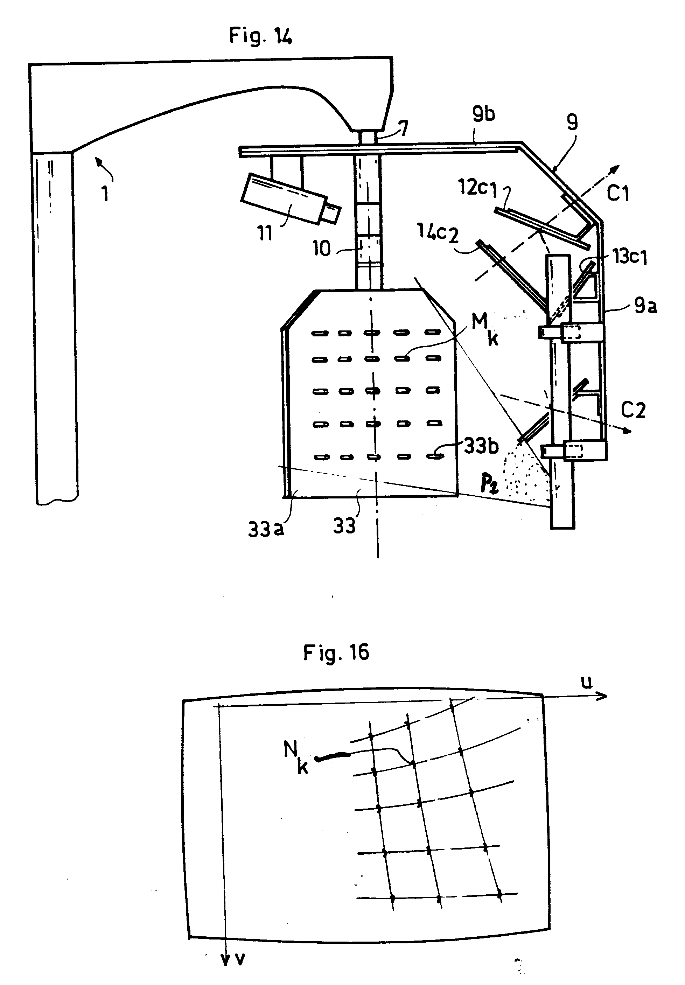

La mire 33 est orientée parallèlement aux plans $P_1$ et $P_2$ des faisceaux des lasers $L_1$, $L_2$, en vue de réaliser deux opérations successives de numérisation décrites ci-après : mire parallèle au plan $P_1$ et mire parallèle au plan $P_2$. Les figures 13 et 14 illustrent la mire dans cette dernière position. Les deux opérations de numérisation sont identiques et une seule sera décrite plus loin (sans mentionner les indices 1 ou 2 du laser concerné).

La mire est éclairée par le laser L et chaque pige 33b, par exemple kième pige notée $M_k$ de coordonnées métriques $x_k$, $z_k$ connues donne une image $N_k$ ($u_k$, $v_k$) sur l'écran (figure 16), $u_k$ désignant la colonne et $v_k$ la ligne où apparaît l'image.

La calibration a pour objectif de définir les fonctions R et T donnant respectivement l'abscisse x et l'ordonnée z de tout point M du plan laser P en fonction de la colonne u et de la ligne v de son image N :

$$M \begin{cases} x = R\,(u,\,v) \\ z = T\,(u,\,v) \end{cases}$$

Dans le cas d'un dispositif linéaire (caméra 11 et électronique associée), on peut démontrer que ces fonctions sont de la forme suivante (fonctions de projection conique, connues en optique) :

$$x = R\,(u, v) = \frac{au + bv + c}{gu + hv + 1}$$
$$z = T\,(u, v) = \frac{du + ev + f}{gu + hv + 1}$$

Les huit inconnues, constituées par les coefficients a, b, c, d, e, f, g, h sont déterminées en appliquant ces équations à au moins quatre piges $M_k$ pour lesquelles $x_k$, $z_k$, $u_k$ et $v_k$ sont connues :

$$M_k \begin{cases} x_k = R\,(u_k,\,v_k) \\ z_k = T\,(u_k,\,v_k) \end{cases} \qquad k \geqslant 4$$

Ce système linéaire est résolu par le micro-ordinateur 27 et les huits coefficients ainsi déterminés sont stockés dans la mémoire 26 pour faire office d'informations représentatives de la géométrie de l'appareil.

En pratique, on choisira un nombre de piges supérieur à 4, notamment compris entre 9 et 25 pour bénéficier de mesures redondantes, de façon à résoudre le système d'équations au sens des moindres carrés en vue de s'affranchir des erreurs de quantification sur $u_k$ et $v_k$ liées à la résolution de la caméra.

Cette numérisation et les calculs qui en découlent sont répétés autant de fois qu'il y a de plans lasers différents et de points de vue caméra différents.

Dans le cas d'un dispositif non linéaire (aberration optique de la caméra, distorsion vidéo, glissement fréquentiel de l'horloge d'échantillonnage du convertisseur 21), on considère que la formulation linéaire précédente est valable localement et que les coefficients a, b, c, d, e, f, g, h sont fonctions du point (u, v).

Dans ces conditions, les fonctions R et T deviennent :

$$x = R\,(u, v) = \frac{a\,(u, v).u + b\,(u, v).v + c\,(u, v)}{g\,(u, v).u + h\,(u, v).v + 1}$$
$$z = T\,(u, v) = \frac{d\,(u, v).u + e\,(u, v).v + f\,(u, v)}{g\,(u, v).u + h\,(u, v).v + 1}$$

Les non-linéarités des coefficients représentent de faibles variations (quelques pour cent) de la fonction de transfert globale du système. Ces coefficients peuvent donc être exprimés sous la forme d'un développement limité, par exemple :

$$a\,(u,\,v) = \sum_{ij\,=\,0}^{n} a_{ij}\,u^i v^j$$

En substituant ces développements dans les fonctions R et T et en factorisant les diverses puissances, on obtient :

$$x = R(u, v) = \frac{\displaystyle\sum_{ij=0}^{n} \alpha_{ij} \, u^i v^j}{1 + \displaystyle\sum_{ij=1}^{n} \gamma_{ij} \, u^i v^j}$$

$$z = T(u, v) = \frac{\displaystyle\sum_{ij=0}^{n} \beta_{ij} \, u^i v^j}{1 + \displaystyle\sum_{ij=1}^{n} \gamma_{ij} \, u^i v^j}$$

On obtient un système linéaire en $\alpha_{ij}$, $\beta_{ij}$, $\gamma_{ij}$ ayant un nombre d'inconnues fonction du degré n de développement, à savoir : $2 + \frac{3n(n+3)}{2}$. On choisira un nombre de piges suffisant pour permettre de résoudre ce système, avec des redondances comme déjà indiqué. Chaque pige donnant deux équations, le nombre de piges minimum sur lequel on applique les équations est : $1 + \frac{3n(n+3)}{4}$.

On a pu constater qu'un développement au degré n = 3 modélise en pratique de façon très satisfaisante les non-linéarités les plus fortes qui peuvent se rencontrer dans les dispositifs utilisés. Pour n = 3, le nombre minimum de piges est de 15 et en pratique ce nombre sera pris compris entre 20 et 30 en vue d'obtenir les redondances précitées.

Il est à noter que le développement sur une base polynomiale $u^i v^j$ peut être remplacé par toute autre base de fonctions (exponentielle, série de Fourier) donnant le cas échéant une meilleure modélisation de certaines distorsions (et permettant ainsi de réduire le degré n de développement nécessaire).

Il est également possible d'effectuer une calibration non linéaire par d'autres méthodes : calibration par zones juxtaposées considérées chacune comme linéaire, calibration par superposition à une calibration linéaire d'une linéarisation préalable de l'image ; cette linéarisation étant effectuée par une caractérisation des non linéarités de l'optique et de l'électronique associée.

## Revendications

1. Procédé de numérisation de la surface d'un objet (ou sujet) tridimensionnel permettant d'engendrer des données numériques représentatives des coordonnées des points de cette surface dans un référentiel tridimensionnel, du type dans lequel :
   - on éclaire la surface de l'objet par au moins deux faisceaux lumineux lamellaires en vue de former des traces lumineuses sur ladite surface selon des surfaces réglées coïncidant ou voisines avec des plans ($P_1$, $P_2$) parallèles à l'axe de rotation (Z) ou passant par cet axe, lesdits faisceaux étant issus de sources ($L_1$, $L_2$) géométriquement décalées le long de la direction de cet axe de rotation de façon que les axes des faisceaux forment avec l'axe de rotation des angles différents ($l_1$, $l_2$),
   - on filme l'objet au moyen d'un dispositif vidéo avec une parallaxe prédéterminée ($P_1$, $P_2$) par rapport aux faisceaux lumineux en vue de délivrer un signal vidéo représentatif des coordonnées des points desdites traces lumineuses, d'au moins deux points de vue différents ($C_1$, $C_2$) géométriquement décalés le long de l'axe de rotation (Z) de façon que les angles ($c_1$, $c_2$) de prises de vue correspondants (considérés par rapport audit axe de rotation) soient différents, en vue de délivrer des signaux vidéo correspondant auxdits points de vue,

- on fait subir un mouvement relatif de rotation autour d'un axe (Z) à l'ensemble dispositif vidéo/faisceaux lumineux par rapport à l'objet de façon à éclairer et filmer toute la surface de l'objet,

- on engendre une information, dite de position angulaire ($\theta$), représentative pour chaque image de la position angulaire correspondante de l'ensemble dispositif vidéo/faisceau lumineux par rapport à l'objet,

- on convertit en données numériques les signaux vidéo représentatifs de chaque image et les informations de position angulaire desdites images ($\theta$),

- on numérise lesdits signaux vidéo en codant dans chaque ligne la position des pixels activés et l'on mémorise ces positions numérisées ($u_1$, $v_1$ ; $u_2$, $v_2$...),

- on réalise, pour chaque image, une partition des positions numérisées en vue de distinguer les positions numérisées correspondant aux traces engendrées par chaque faisceau,

- on engendre à partir des informations de position angulaire liées à chaque image ($\theta$) et d'informations ($p_1$, $p_2$, $c_1$, $c_2$ ; a-h ; $\alpha_{ij}$, $\beta_{ij}$, $\gamma_{ij}$) représentatives de la géométrie de l'ensemble dispositif vidéo/faisceaux lumineux, des informations dites spécifiques, attachées à chaque trace,

- et, pour chaque image, on associe aux positions numérisées correspondant à chaque trace ($u_1$, $v_1$ ou $u_2$, $v_2$...) les informations spécifiques attachées à la trace considérée,

procédé caractérisé en ce que les informations représentatives de la géométrie de l'ensemble vidéo/faisceaux lumineux sont déterminées par une procédure de calibration consistant :

∗ à réaliser préalablement la numérisation d'une mire de calibration (33) substituée à l'objet, ladite mire présentant des reliefs de géométrie et de dimensions connues préalablement mémorisées,

∗ et à calculer des fonctions x = R (u, v), z = T (u, v) donnant les coordonnées métriques des points de chaque plan laser en fonction des colonnes et lignes de l'image correspondante, à partir des résultats de la numérisation précitée, les informations spécifiques attachées à chaque trace étant obtenues par l'application de ces fonctions.

2. Procédé selon la revendication 1, dans lequel, en combinaison :

- l'on filme l'objet de deux points de vue ($C_1$, $C_2$) situés dans un plan médian (M) contenant l'axe de rotation (Z) ou au voisinage de celui-ci, à des hauteurs différentes pour donner un angle de prise de vue en plongée ($c_1$) et l'autre en contreplongée ($c_2$),

- l'on éclaire la surface de l'objet par deux faisceaux lumineux distincts issus de deux sources ($L_1$, $L_2$), situées de part et d'autre du plan médian (M) à des hauteurs différentes pour éclairer, l'une en plongée ($l_1$), l'autre en contreplongée ($l_2$).

3. Procédé selon la revendication 2, caractérisé en ce que l'on filme l'objet des deux points de vue ($C_1$, $C_2$) de sorte que les champs de prises de vue soient sensiblement disjoints et correspondent, chacun, au demi-espace ($E_1$, $E_2$) situé d'un côté et de l'autre du plan médian (M), de façon à filmer de chaque point de vue la trace d'un seul faisceau lumineux.

4. Procédé selon la revendication 1, dans lequel, en combinaison :

- l'on éclaire la surface de l'objet par deux faisceaux lumineux situés dans un plan médian (M') contenant l'axe de rotation (Z') ou situés au voisinage d'un tel plan, à partir de deux sources ($L'_1$, $L'_2$) décalées en hauteur, de façon à éclairer, l'une en plongée ($l'_1$), l'autre en contreplongée ($l'_2$),

- l'on filme l'objet de deux points de vue ($C'1$, $C'2$) situés de part et d'autre du plan médian (M') précité, à des hauteurs différentes pour donner un angle de prise de vue en plongée ($c_1$) et en contreplongée ($c_2$).

5. Procédé selon la revendication 4, caractérisé en ce que l'on filme l'objet des deux points de vue ($C'_1$, $C'_2$) de façon que les champs de prises de vue se recouvrent au niveau de l'objet et contiennent la trace des deux faisceaux lumineux.

6. Procédé selon l'une des revendications 1, 2, 3, 4 ou 5, dans lequel l'on filme l'objet au moyen d'une caméra unique (11) par réflexion sur deux jeux de miroirs ($12c_1$, $13c_1$ ; $14c_2$, $15c_2$), géométriquement agencés pour engendrer les deux points de vue ($C_1$, $C_2$) précités et conditionner des chemins optiques de longueur voisine, en vue de délivrer un signal vidéo unique représentatif de deux demi-images correspondant à chaque point de vue.

7. Procédé selon la revendication 6, prise en dépendance de la revendication 3, caractérisé en ce que l'on numérise le signal vidéo en codant dans chaque ligne la position des pixels activés et l'on réalise la partition des positions numérisées en fonction de leur valeur par rapport à une valeur médiane correspondant à la position de l'axe de rotation (Z) dans l'image.

8. Procédé selon l'une des revendications 1, 2, 3, 4, ou 5, caractérisé en ce que l'on filme l'objet au moyen de deux caméras, chacune disposée pour observer selon l'un des points de vue ($C'_1$, $C'_2$) précités, en vue de délivrer deux signaux vidéo représentatifs de deux images correspondant aux deux points de vue.

9. Procédé selon la revendication 8, prise en dépendance de la revendication 4, caractérisé en ce que l'on multiplexe les deux signaux vidéo en vue de leur numérisation et l'on réalise, pour chaque image, la partition des positions numérisées en fonction de la caméra dont est issue l'image considérée.

10. Procédé selon l'une des revendications 1, 2, 3, 4, 5, 6, 7, 8 ou 9, dans lequel l'on éclaire la surface de l'objet par des sources définies par deux dispositifs lasers distincts ($16l_1$, $17l_2$).

11. Procédé selon l'une des revendications 1 à 10, dans lequel les informations représentatives de la géométrie de l'ensemble vidéo/faisceaux lumineux sont constituées par des distances et des angles ($p_1$, $p_2$, $c_1$, $c_2$) préalablement mesurés et stockés dans une mémoire (26), les informations spécifiques attachées à chaque trace étant les coordonnées des points desdites traces calculées par des calculs trigonométriques.

12. Procédé selon la revendication 1, caractérisé en ce que ladite mire de calibration est constituée par une plaque (33a) portant sur une face un maillage de piges (33b), la numérisation étant effectuée pour chaque plan laser en disposant ladite plaque (33a) parallèlement au plan du faisceau lumineux de façon que les piges interceptent ledit faisceau.

13. Procédé selon l'une des revendications 1 ou 12, caractérisé en ce que l'on calcule des fonctions linéaires de la forme $x = R(u, v) = \dfrac{au + bv + c}{gu + hv + 1}$ et $z = T(u, v) = \dfrac{du + ev + f}{gu + hv + 1}$, les coefficients (a-h) étant déterminés en appliquant ces équations à au moins quatre points des reliefs de la mire, pour lesquels le quadruplet (x, z, u, v) est connu et mémorisé, et en résolvant le système linéaire d'équations obtenu.

14. Procédé selon l'une des revendications 1 ou 12, caractérisé en ce que l'on calcule des fonctions non linéaires de la forme

$$x = R(u, v) = \frac{\displaystyle\sum_{ij=0}^{n} \alpha_{ij}\, u^i v^j}{1 + \displaystyle\sum_{ij=1}^{n} \gamma_{ij}\, u^i v^j}$$

$$z = T(u, v) = \frac{\displaystyle\sum_{ij=0}^{n} \beta_{ij}\, u^i v^j}{1 + \displaystyle\sum_{ij=1}^{n} \gamma_{ij}\, u^i v^j}$$

les coefficients $\alpha_{ij}$, $\beta_{ij}$, $\gamma_{ij}$ étant déterminés en appliquant ces équations à un nombre de points de reliefs de la mire, au moins égal à $1 + \dfrac{3n(n+3)}{4}$, pour lesquels le quadruplet (x, z, u, v) est connu et mémorisé, et en résolvant le système linéaire d'équations obtenu.

15. Procédé selon l'une des revendications 1 à 14, caractérisé en ce que l'objet est fixe et traversé par l'axe de rotation (Z), et l'ensemble dispositif vidéo/faisceaux lumineux est amené à tourner autour de l'axe de rotation (Z).

16. Appareil de relevé tridimensionnel de la surface d'un objet (ou sujet) en vue de délivrer un signal vidéo représentatif des coordonnées des points de cette surface, du type comprenant :
   - un système d'éclairage de l'objet ($16l_1$, $17l_2$), adapté pour définir deux sources ($L_1$, $L_2$) géométriquement décalées le long de la direction de l'axe de rotation, en vue d'émettre vers la surface de l'objet deux faisceaux lamellaires distincts, s'étendant dans des plans parallèles à l'axe de rotation (Z) ou passant par celui-ci, les axes desdits faisceaux formant avec ledit axe de rotation (Z) deux angles différents ($l_1$, $l_2$),
   - un dispositif vidéo (11, $12c_1$, $13c_1$, $14c_1$, $15c_2$) mécaniquement solidaire du système d'éclairage, apte à filmer l'objet avec une parallaxe prédéterminée par rapport aux faisceaux du système d'éclairage, adapté pour capter des images de l'objet de deux points de vue différents ($C_1$, $C_2$) géométriquement décalés le long de l'axe de rotation (Z) de façon que les deux angles de prises de vue correspondants ($c_1$, $c_2$) considérés par rapport audit axe de rotation soient différents, en vue d'être apte à délivrer un ou des signaux vidéo correspondant auxdits points de vue,
   - des moyens (3, 10) de positionnement de l'objet sur la trajectoire des faisceaux du système d'éclairage et dans le champ du dispositif vidéo,
   - des moyens (4-7) de déplacement en rotation relative de l'ensemble système d'éclairage/dispositif vidéo par rapport à l'objet autour d'un axe de rotation (Z),
   <u>appareil caractérisé en ce que</u> le support tournant (9) est doté d'un organe de centrage (10), situé dans le prolongement de l'arbre de sortie (7) et adapté pour assurer un positionnement de l'objet, et en ce que l'organe de centrage (10) est adapté pour recevoir des embouts amovibles d'au moins deux types, l'un (10a, 34) adapté pour venir en appui sur l'objet à numériser, l'autre (36) adapté pour supporter et centrer une mire de calibration (33) associée à l'appareil, et en ce qu'il comprend des moyens de calibration, propres à numériser l'image de la surface de ladite mire et calculer, à partir des résultats de cette numérisation, des fonctions x = R (u, v) et z = T (u,v) donnant les coordonnées métriques des points de chaque plan laser en fonction des colonnes et lignes de l'image correspondante, les informations spécifiques attachées à chaque trace étant ensuite obtenues par application de ces fonctions.

17. Appareil selon la revendication 16 dans lequel, en combinaison :
   - le dispositif vidéo comprend une caméra vidéo (11) spécifiquement sensible à une longueur d'onde prédéterminée, et deux jeux de miroirs ($12c_1$, $13c_1$ ; $14c_2$, $15c_2$...) géométriquement agencés pour engendrer deux points de vue ($C_1$, $C_2$) situés dans un plan médian (M) contenant l'axe de rotation (Z), l'un des jeux donnant un angle de prise de vue en plongée, l'autre en contre-plongée,
   - le système d'éclairage comprend deux dispositifs lasers lamellaires ($16l_1$, $17l_1$) de longueur d'onde accordée à celle de la caméra, agencés pour définir des sources ($L_1$, $L_2$) disposées latéralement d'un côté et de l'autre du plan médian (M) précité, à des hauteurs différentes pour éclairer, l'une en plongée, l'autre en contre-plongée.

18. Appareil selon la revendication 17, caractérisé en ce que les jeux de miroirs ($12c_1$, $13c_1$ ; $14c_2$, $15c_2$) sont agencés pour couvrir des champs de prises de vue sensiblement disjoints correspondant respectivement aux demi-espaces ($E_1$, $E_2$) situés d'un côté et de l'autre du plan médian (M), le jeu de miroirs ($12c_1$, $13c_1$) donnant l'angle de prise de vue en plongée ($c_1$) couvrant le demi-espace ($E_1$) situé du côté de la source laser éclairant en plongée ($L_1$), et réciproquement pour la contre-plongée.

19. Appareil selon l'une des revendications 17 ou 18, caractérisé en ce qu'il comprend une potence (1), un moteur électrique (4) associé à un réducteur (5) portés par ladite potence, un système de transmission (6) associé audit réducteur et possédant un arbre de sortie (7) colinéaire à l'axe de rotation (Z), et un support tournant (9) porté par l'arbre de sortie, la caméra vidéo (11), les jeux de miroirs ($12c_1$, $13c_1$ ; $14c_2$, $15c_2$) et les dispositifs lasers ($16l_1$, $17l_2$) étant assujettis audit support tournant (9).

20. Appareil selon l'une des revendications 16 à 19, dans lequel un capteur de position angulaire (8) est associé aux moyens de déplacement en rotation (4-7), ledit capteur étant adapté pour délivrer un signal représentatif de la position angulaire relative de l'ensemble système d'éclairage/dispositif vidéo par rapport à l'objet.

21. Appareil selon la revendication 20, prise en dépendance de la revendication 19, caractérisé en ce que le capteur de position angulaire (8) comprend un générateur d'impulsions électriques de fréquence proportionnelle à la vitesse de rotation, couplé à l'arbre du moteur électrique.

22. Appareil selon la revendication 16, caractérisé en ce que la mire de calibration comprend une plaque (33a) dotée d'un maillage de piges (33b).

23. Appareil selon l'une des revendications 19 à 22, caractérisé en ce que le support tournant (9) possède, d'une part, une extension frontale (9a) située à l'avant de l'organe de centrage (10) et portant les jeux de miroirs ($12c_1$, $13c_1$ ; $14c_2$, $15c_2$) et les dispositifs lasers ($16l_1$, $17l_2$), d'autre part, une extension supérieure (9b) portant la caméra (11), l'organe de centrage (10) étant ajouré pour dégager le chemin optique entre caméra et jeux de miroirs.

24. Appareil selon l'une des revendications 16 à 23, adapté pour effectuer le relevé d'une tête ou d'un buste humain.

## Patentansprüche

1. Verfahren zum Digitalisieren der Oberfläche eines dreidimensionalen Gegenstandes (oder Inhaltes), mit dem digitale Daten erzeugbar sind, die die Koordinaten der Punkte dieser Oberfläche in einem dreidimensionalen Bezugssystem repräsentieren, in dem:

- die Oberfläche des Gegenstandes durch zumindest zwei lamellenförmige Strahlenbündel beleuchtet wird, um Lichtspuren auf der Oberfläche gemäß den Oberflächen, die mit zu der Drehachse (Z) parallelen Ebenen ($P_1$, $P_2$) zusammenfallend oder benachbart oder durch diese Achse hindurchgehend ausgerichtet sind, zu erzeugen, wobei die Strahlenbündel von Quellen ($L_1$, $L_2$) ausgegeben werden, die in Längsrichtung dieser Drehachse geometrisch versetzt liegen, so daß die Achsen der Strahlenbündel mit der Drehachse unterschiedliche Winkel ($l_1$, $l_2$) bilden,

- der Gegenstand mittels einer Videovorrichtung mit einer vorbestimmten Parallaxe ($P_1$, $P_2$) in bezug auf die Strahlenbündel gefilmt wird, um ein Videosignal auszugeben, das die Koordinaten der Punkte der Lichtspuren repräsentiert, wobei zumindest zwei unterschiedliche Blickpunkte ($C_1$, $C_2$) entlang der Drehachse (Z) geometrisch versetzt liegen, so daß die winkel ($c_1$, $c_2$) der entsprechenden Aufnahmen (in bezug auf die Drehachse betrachtet) unterschiedlich sind, um die Videosignale entsprechend den Blickpunkten zu liefern,

- die gesamte Video-/Strahlenbündelvorrichtung einer relativen Drehbewegung um eine Achse (Z) in bezug zu dem Gegenstand unterzogen wird, um so die gesamte Oberfläche des Gegenstandes zu beleuchten und zu filmen,

- eine die Winkelposition ($\Theta$) wiedergebende Information erzeugt wird, welche repräsentativ ist für jedes Bild der entsprechenden Winkelposition der Video/Strahlenbündelvorrichtung in bezug zu dem Gegenstand,

- die Videosignale, die für jedes Bild repräsentativ sind, und die Informationen über die Winkelpositionen der Bilder ($\Theta$) in digitale Daten umgewandelt werden,

- die Videosignale digitalisiert werden, indem in jeder Zeile die Position der aktivierten Pixel kodiert wird und diese digitalisierten Positionen ($u_1$, $v_1$; $u_2$, $v_2$...) gespeichert werden,

- für jedes Bild eine Unterteilung der digitalisierten Positionen durchgeführt wird, um die den durch jedes Strahlenbündel erzeugten Spuren entsprechenden digitalisierten Positionen zu unterscheiden,

- aus den Informationen zur Winkelposition, die mit jedem Bild ($\Theta$) verbunden sind, und aus den Informationen ($p_1$, $p_2$, $c_1$, $c_2$; a-h; $\alpha_{ij}$, $\beta_{ij}$, $\gamma_{ij}$), die die Geometrie der gesamten Video-/Strahlenbündelvorrichtung repräsentieren, spezifische Informationen erzeugt werden, die jeder Spur anhaften,

- und, für jedes Bild aus den digitalisierten Positionen, entsprechend jeder Spur ($u_1$, $v_1$, $v_1$ oder $u_2$, $v_2$ ...), die an der betrachteten Spur anhaftenden Informationen zugeordnet werden,

**dadurch gekennzeichnet,**

daß die Informationen, die die Geometrie der gesamten Video-/Strahlenbündelvorrichtung repräsentieren, durch ein Kalibrierverfahren bestimmt werden, bestehend aus:

- zuerst die Digitalisierung eines Kalibrier-Testobjektes (33) anstelle eines Gegenstandes durchzuführen, wobei das Testobjekt vorher gespeicherte geometrische Reliefs und bekannte Dimensionen aufweist,
- und, die Funktionen x = R (u, v), z = T (u, v) zu berechnen, die die metrischen Koordinaten der Punkte jeder Laserebene als Funktion der Spalten und Zeilen des entsprechenden Bildes liefern, wobei aus den Ergebnissen der vorerwähnten Digitalisierung die spezifischen Informationen, die an jeder Spur anhaften, durch Anwendung dieser Funktionen erhalten werden.

2. Verfahren nach Anspruch 1, in dem in Kombination:
- der Gegenstand von zwei Blickpunkten ($C_1$, $C_2$) gefilmt wird, die in einer die Drehachse (Z) enthaltende Mittelebene (M) oder in Nachbarschaft von dieser in unterschiedlichen Höhen liegen, um einen abtauchenden Aufnahmewinkel ($c_1$) und den anderen von unten auftauchend ($c_2$) zu liefern,
- die Oberfläche des Gegenstandes durch zwei scharfe Strahlenbündel beleuchtet wird, die von zwei Quellen ($L_1$, $L_2$) ausgegeben werden, die beiderseits der Mittelebene (M) an unterschiedlichen Höhen liegen, um einereseits abtauchend ($l_1$), andererseits auftauchend ($l_2$) zu beleuchten.

3. Verfahren nach Anspruch 2,
**dadurch gekennzeichnet,**
daß der Gegenstand von zwei Blickpunkten ($C_1$, $C_2$) aus gefilmt wird, so daß die Kamerabereiche genau getrennt sind und jeweils einem Halbraum ($E_1$, $E_2$) entsprechen, der an der einen und der anderen Seite der Mittelebene (M) liegt, um so von jedem Blickpunkt aus die Spur von einem einzigen Strahlenbündel zu filmen.

4. Verfahren nach Anspruch 1, in dem in Kombination:
- die Oberfläche des Gegenstandes aus zwei in der Höhe versetzt liegenden Quellen ($L'_1$, $L'_2$) durch zwei Strahlenbündel beleuchtet wird, die in einer die Drehachse (Z') enthaltenen Mittelebene (M') liegen, oder die einer solchen Ebene benachbart liegen, um einerseits abtauchend ($l'_1$), andererseits auftauchend ($l'_2$) zu beleuchten,
- der Gegenstand von zwei Blickpunkten ($C'_1$, $C'_2$) gefilmt wird, die beiderseits der vorbeschriebenen Mittelebene (M') an unterschiedlichen Höhen liegen, um einen abtauchenden ($c_1$) und einen auftauchenden ($c_2$) Aufnahmewinkel zu liefern.

5. Verfahren nach Anspruch 4,
**dadurch gekennzeichnet,**
daß der Gegenstand von zwei Blickpunkten ($C'_1$, $C'_2$) gefilmt wird, so daß sich die Kamerabereiche auf dem Niveau des Gegenstandes überdecken und die Spur der zwei Strahlenbündel enthalten.

6. Verfahren nach einem der Ansprüche 1, 2, 3, 4 oder 5, in dem der Gegenstand zumindest von einer einzigen Kamera (11) gefilmt wird, durch Reflexion über zwei Spiegelgruppen (12 $c_1$, 13 $c_1$; 14 $c_2$, 15 $c_2$,), die geometrisch angeordnet sind, um die zwei vorbeschriebenen Blickpunkte ($C_1$, $C_2$) zu erzeugen und die Lichtwege auf die benachbarte Länge zu konditionieren, um ein einziges Videosignal zu liefern, das zwei Halbbilder repräsentiert entsprechend jedem Blickpunkt.

7. Verfahren nach Anspruch 6, gefaßt in Abhängigkeit von Anspruch 3,
**dadurch gekennzeichnet,**
daß das Videosignal digitalisiert wird, indem in jeder Zeile die Position der aktivierten Pixel kodiert wird, und daß die Einteilung der digitalisierten Positionen als Funktion ihres Wertes in bezug auf einen Medianwert, der der Position der Drehachse (Z) in dem Bild entspricht, durchgeführt wird.

8. Verfahren nach einem der Ansprüche nach 1, 2, 3, 4 oder 5,
**dadurch gekennzeichnet,**
daß der Gegenstand mittels zwei Kameras gefilmt wird, wobei jede so angeordnet ist, daß sie gemäß einer der vorbeschriebenen Blickpunkte ($C'_1$, $C'_2$) beobachten kann, um zwei Videosignale zu liefern, die zwei den zwei Blickpunkten entsprechende Bilder repräsentieren.

9. Verfahren nach Anspruch 8, gefaßt in Abhängigkeit von Anspruch 4,
**dadurch gekennzeichnet,**
daß die zwei Videosignale multiplext werden hinsichtlich ihrer Digitalisierung, und daß für jedes Bild die Einteilung der digitalisierten Positionen in Abhängigkeit von der Kamera, von der das betrachtete Bild ausgegeben wird, durchgeführt wird.

10. Verfahren nach einem der Ansprüche 1, 2, 3, 4, 5, 6, 7, 8 oder 9, in dem die Oberfläche des Gegenstandes durch definierte Quellen mit zwei scharfen Laservorrichtungen ($16l_1$, $17l_2$) beleuchtet wird.

11. Verfahren nach einem der Ansprüche 1-10, in dem die Informationen, die die Geometrie der gesamten Video/Strahlenbündelvorrichtung repräsentieren, aus den zuvor gemessenen Abständen und winkeln ($p_1$, $p_2$, $c_1$, $c_2$) gebildet und in einem Speicher (26) gespeichert werden, wobei die an jeder Spur anhaftenden spezifischen Informationen die Punktkoordinaten dieser Spuren sind, die durch trigonometrische Berechnungen berechnet werden.

12. Verfahren nach Anspruch 1,
    **dadurch gekennzeichnet,**
    daß das Kalibrier-Testobjekt aus einer Platte (33a) gebildet wird, die auf einer Fläche ein Netz aus Peilstäben (33b) trägt, wobei die Digitalisierung für jede Laserebene bewirkt wird, indem die Platte (33a) parallel zur Fläche des Strahlenbündels angeordnet wird, so daß die Peilstäbe das Strahlenbündel abfangen.

13. Verfahren nach einem der Ansprüche 1 oder 12,
    **dadurch gekennzeichnet,**
    daß die Linearfunktionen aus der Formel $x = R(u, v) = \dfrac{au + bv + c}{gu + hv + 1}$ und $z = T(u, v)$

    $= \dfrac{du + ev + f}{gu + hv + 1}$ berechnet werden, wobei die Koeffizienten (a-h) bestimmt werden, indem diese Gleichungen an zumindest vier Reliefpunkten des Testobjektes angewendet werden, wobei für diese das Quadrupel (x, z, u, v) erkannt und gespeichert wird, und indem das erhaltene Linearsystem der Gleichungen aufgelöst wird.

14. Verfahren nach einem der Ansprüche 1 oder 12,
    **dadurch gekennzeichnet,**
    daß die nicht-linearen Funktionen nach der Formel

$$x = R(u, v) = \frac{\displaystyle\sum_{ij = 0}^{n} \alpha_{ij}\, u^i v^j}{1 + \displaystyle\sum_{ij = 1}^{n} \gamma_{ij}\, u^i v^j}$$

$$z = T(u, v) = \frac{\displaystyle\sum_{ij = 0}^{n} \beta_{ij}\, u^i v^j}{1 + \displaystyle\sum_{ij = 1}^{n} \gamma_{ij}\, u^i v^j}$$

berechnet werden, wobei die Koeffizienten $\alpha_{ij}$, $\beta_{ij}$, $\gamma_{ij}$ bestimmt werden, indem diese Gleichungen auf eine Anzahl von Reliefpunkten des Testobjektes angewendet werden, auf zumindest gleich $1 + \dfrac{3n(n + 3)}{4}$, für die das Quadrupel (x, z, u, v) erkannt und gespeichert wird, und indem das erhaltene Linearsystem der Gleichungen aufgelöst wird.

15. Verfahren nach einem der Ansprüche 1-14,
    **dadurch gekennzeichnet,**
    daß der Gegenstand festliegt und von der Drehachse (Z) durchquert wird, und die gesamte Video/Strahlenbündelvorrichtung veranlaßt wird, sich um die Drehachse (Z) zu drehen.

16. Vorrichtung zum dreidimensionalen Aufnehmen der Oberfläche eines Gegenstandes (oder Inhaltes), um ein Videosignal zu liefern, das die Koordinaten der Punkte dieser Oberfläche repräsentiert, mit:
    - einer Anlage zur Beleuchtung des Gegenstandes ($16l_1$, $17l_2$), die so ausgebildet ist, daß zwei geometrisch in Längsrichtung der Drehachse versetzte Quellen ($L_1$, $L_2$) definiert sind, um auf die Oberfläche des Gegenstandes zwei scharfe Strahlen, die sich in parallelen Ebenen zur Drehachse (Z) ausbreiten oder durch diese hindurchgehen, auszusenden, wobei die Achsen der Strahlenbündel mit der Drehachse (Z) zwei unterschiedliche winkel ($l_1$, $l_2$) bilden,
    - einer mit der Beleuchtungsanlage mechanisch einstückigen Videovorrichtung (11, $12c_1$, $13c_1$, $14c_1$, $15c_2$), geeignet, den Gegenstand mit einer in bezug auf die Strahlenbündel der Beleuchtungsanlage vorbestimmten Parallaxe zu filmen, die so ausgebildet ist, daß die Bilder des Gegenstandes von zwei unterschiedlichen Blickpunkten ($C_1$, $C_2$) aufgenommen werden, die entlang der Drehachse (Z) so geometrisch versetzt liegen, daß die zwei entsprechenden Blickwinkel ($c_1$, $c_2$) in bezug zur Drehachse betrachtet unterschiedlich sind, um so geeignet zu sein, ein oder mehrere Videosignale entsprechend der Blickpunkte zu liefern,
    - Mitteln (3, 10) zum Positionieren des Gegenstandes auf der Strahlenbahn der Beleuchtungsanlage und im Feld der Videovorrichtung,
    - Mitteln (4-7) zum Verstellen der gesamten Beleuchtungsanlage/Videovorrichtung durch eine Relativdrehung um eine Drehachse (Z) in bezug auf den Gegenstand,
    **dadurch gekennzeichnet,**
    daß die Drehstütze (9) mit einer Zentriereinrichtung (10) versehen ist, die auf der Verlängerung der Abtriebswelle (7) sitzt und so ausgebildet ist, daß eine Positionierung des Gegenstandes sichergestellt ist, und daß die Zentriereinrichtung (10) so ausgebildet ist, daß zumindest zwei Arten von abnehmbaren Ansatzstücken aufgenommen sind, wobei das eine (10a, 34) zum Aufstützen auf den zur digitalisierenden Gegenstand ausgebildet ist, und das andere (36) zum Abstützen und Zentrieren eines der Vorrichtung zugeordneten Kalibrier-Testobjektes (33) ausgebildet ist, und daß sie Kalibriereinrichtungen umfaßt, die geeignet sind, das Oberflächenbild des Testobjektes zu digitalisieren und aus den Ergebnissen der Digitalisierung die Funktionen x = R (u, v) und z = T (u, v) zu berechnen, die die metrischen Koordinaten der Punkte jeder Laserfläche in Abhängigkeit von den Spalten und Zeilen des entsprechenden Bildes wiedergeben, wobei die an jeder Spur anhaftenden spezifischen Informationen daraufhin durch Anwendung dieser Funktionen erhalten werden.

17. Vorrichtung nach Anspruch 16, in der in Kombination:
    - die Videovorrichtung eine Videokamera (11), die besonders empfindlich auf eine vorbestimmte Wellenlänge ist, und zwei Spiegelgruppen ($12c_1$, $13c_1$; $14c_2$, $15c_2$ ...), die geometrisch angeordnet sind, um zwei Blickpunkte ($C_1$, $C_2$) zu erzeugen, die in einer die Drehachse (Z) enthaltenen Mittelebene (M) liegen, wobei eine der Gruppen einen abtauchenden, die andere einen auftauchenden Blickwinkel liefert, umfaßt,
    - die Beleuchtungsanlage zwei lamellenförmige Laservorrichtungen ($16l_1$, $17l_1$) mit einer Wellenlänge, die mit der der Kamera übereinstimmt, umfaßt, welche so angeordnet sind, daß die Quellen ($L_1$, $L_2$), die seitlich auf der einen und anderen Seite der vorbeschriebenen Mittelebene (M) an unterschiedlichen Höhen angeordnet sind, um einerseits abtauchend, andererseits auftauchend zu leuchten.

18. Vorrichtung nach Anspruch 17,
    **dadurch gekennzeichnet,**
    daß die Spiegelgruppen ($12c_1$, $13c_1$; $14c_2$, $15c_2$) so ausgerichtet sind, daß sie Kamerabereiche, die genau getrennt sind, jeweils entsprechend den auf der einen und der anderen Seite der Mittelebene (M) gelegenen Halbräumen ($E_1$, $E_2$), abdecken, wobei die Spiegelgruppe ($12c_1$, $13c_1$) einen abtauchenden Blickwinkel ($c_1$), der den auf der Seite der abtauchend leuchtenden Laserquelle ($L_1$) liegenden Halbraum ($E_1$) abdeckt, liefert, und umgekehrt für den auftauchenden.

19. Vorrichtung nach einem der Ansprüche 17 oder 18,

**dadurch gekennzeichnet,**
daß diese einen Träger (1), einen Elektromotor (4), der mit einem Untersetzungsgetriebe (5) verbunden ist, die von dem Träger getragen werden, eine Übertragungseinrichtung (6), die mit dem Untersetzungsgetriebe verbunden ist und eine mit der Drehachse (Z) co-lineare Abtriebswelle (7) aufweist, und eine Drehstütze (9) umfaßt, die auf der Abtriebswelle sitzt, wobei die Videokamera (11), die Spiegelgruppen ($12c_1$, $13c_1$; $14c_2$, $15c_2$) und die Laservorrichtungen ($16l_1$, $17l_2$) abhängig von der Drehstütze (9) sind.

20. Vorrichtung nach einem der Ansprüche 16-19, in der ein Meßfühler für die Winkelposition (8) mit einer Dreh-Verstelleinrichtung (4-7) verbunden ist, wobei der Meßfühler so ausgebildet ist, daß er ein Signal liefert, daß die relative Winkelposition der gesamten Beleuchtungsanlage/Videovorrichtung in bezug zu dem Gegenstand repräsentiert.

21. Vorrichtung gemäß Anspruch 20, gefaßt in Abhängigkeit von Anspruch 19,
    **dadurch gekennzeichnet,**
    daß der Meßfühler der Winkelposition (8) einen elektrischen Taktgeber mit einer der Drehgeschwindigkeit proportionalen Frequenz umfaßt, der an die welle des Elektromotors gekoppelt ist.

22. Vorrichtung nach Anspruch 16,
    **dadurch gekennzeichnet,**
    daß das Kalibrier-Testobjekt eine Platte (33a) umfaßt, die mit einem Netz von Peilstäben (33b) versehen ist.

23. Vorrichtung nach einem der Ansprüche 19-22,
    **dadurch gekennzeichnet,**
    daß die Drehstütze (9) einerseits eine stirnseitige Erstreckung(9a), die vor der Zentriereinrichtung (10) liegt und die Spiegelgruppen ($12c_1$, $13c_1$; $14c_2$, $15c_2$) und die Laservorrichtungen ($16l_1$, $17l_2$) trägt, und andererseits eine obenliegende Erstreckung (9b) aufweist, die die Kamera (11) trägt, wobei die Zentriereinrichtung (10) durchbrochen ist, um den optischen weg zwischen Kamera und Spiegelgruppen freizumachen.

24. Vorrichtung nach einem der Ansprüche 16-23, so ausgebildet, daß die Aufnahme eines Kopfes oder eines menschlichen Oberkörpers durchführbar ist.

## Claims

1. Method of digitization of the surface of a three-dimensional object (or subject) permitting the generation of digital data representing coordinates of the points of this surface in a three-dimensional reference system, of the type wherein
   - the surface of the object is illuminated by at least two flat light beams in order to form light traces of the said surface along adjusted surfaces coinciding with or adjacent to planes ($P_1$, $P_2$) parallel to the axis of rotation (Z) or passing through this axis, the said beams being emitted from sources ($L_1$, $L_2$) geometrically displaced in the direction of this axis of rotation in such a way that the axes of the beams form different angles with the axis of rotation ($l_1$, $l_2$);
   - the object is filmed by means of a video device with a predetermined parallax ($p_1$, $p_2$) with respect to the light beams, in order to deliver a video signal representing the co-ordinates of the points of the said light traces, from at least two different viewpoints ($C_1$, $C_2$) geometrically displaced along the axis of rotation (Z) in such a way that the corresponding filming angles ($c_1$, $c_2$) (considered with respect to the said axis of rotation) are different, in order to deliver video signals corresponding to the said viewpoints;
   - the video device and light beam assembly is made to undergo a relative rotary movement about an axis (Z) with respect to the object in such a way as to illuminate and film the whole surface of the object;
   - a data element, known as the angular position element ($\theta$) is generated to represent, for each image, the corresponding angular position of the video and light beam assembly with respect to the object;
   - the video signals representing each image and the angular position elements of the said images ($\theta$) are converted into digital data;
   - the said video signals are digitized by coding the positions of the activated pixels in each line, and these digitized positions ($u_1$, $v_1$; $u_2$, $v_2$...) are stored;
   - for each image, the digitized positions are separated in order to distinguish the digitized positions corresponding to the traces generated by each beam;

- data elements known as specific elements, attached to each trace, are generated from angular position elements associated with each image ($\theta$) and from data elements ($p_1$, $p_2$, $c_1$, $c_2$; a-h; $\alpha_{iJ}$, $\beta_{iJ}$, $\gamma_{ij}$) representing the geometry of the video and light beam assembly;

- and, for each image, the specific data elements attached to the trace concerned are associated with the digitized positions corresponding to each trace ($u_1$, $v_1$; $u_2$, $v_2$...);

the method being characterized in that the data representing the geometry of the video and light beam assembly are determined by a calibration procedure comprising

∗ the preliminary digitization of a calibration pattern (33) substituted for the object, the said pattern having relief areas with previously stored known geometry and dimensions; and

∗ the calculation of the functions x = R (u, v), z = T (u, v) giving the metrical co-ordinates of the points of each laser plane as a function of the columns and lines of the corresponding image, from the results of the aforesaid digitization, the specific data attached to each trace being obtained by the application of these functions.

2. A method according to claim 1, wherein, in combination,

- the object is filmed from two viewpoints ($C_1$, $C_2$) situated in a median plane (M) containing the axis of rotation (Z) or in the vicinity of this axis, at different heights, to give a high filming angle ($c_1$) and a low filming angle ($c_2$);

- the surface of the object is illuminated by two separate light beams emitted from two sources ($L_1$, $L_2$) situated on opposite sides of the median plane (M) at different heights so that one provides high-angle illumination ($I_1$) and the other provides low-angle illumination ($I_2$).

3. A method according to claim 2, characterized in that the object is filmed from two viewpoints ($C_1$, $C_2$) in such a way that the filming areas are appreciably separated and each corresponds to a half-space ($E_1$, $E_2$) situated on one and other side of the median plane (M), in such a way that the trace of a single light beam is filmed from each viewpoint.

4. A method according to claim 1, wherein, in combination,

- the surface of the object is illuminated by two light beams situated in a median plane (M′) containing the axis of rotation (Z′) or situated in the vicinity of such a plane, from two sources (L′$_1$, L′$_2$) displaced with respect to height in such a way that one provides high-angle illumination ($I_1$) and the other provides low-angle illumination ($I_2$); and

- the object is filmed from two viewpoints ($C_1$, $C_2$) situated on opposite sides of the aforesaid median plane (M′) at different heights, to give a high filming angle ($c_1$) and a low filming angle ($c_2$).

5. A method according to claim 4, characterized in that the object is filmed from two viewpoints ($C_1$, $C_2$) in such a way that the filming areas overlap at the position of the object and contain the traces of the two light beams.

6. A method according to one of claims 1, 2, 3, 4 or 5, wherein the object is filmed with a single camera (11) by reflection from two sets of mirrors ($12c_1$, $13c_1$; $14c_2$, $15c_2$), geometrically arranged to generate the aforesaid two viewpoints ($C_1$, $C_2$) and to provide optical paths of similar length in order to deliver a single video signal representing two half images corresponding to the two viewpoints.

7. A method according to claim 6, when dependent on claim 3, characterized in that the video signal is digitized by coding the positions of the activated pixels in each line and in that the digitized positions are divided according to their value with respect to a median value corresponding to the position of the axis of rotation (Z) in the image.

8. A method according to one of claims 1, 2, 3, 4 or 5, characterized in that the object is filmed with two cameras, each disposed to observe from one of the aforesaid two viewpoints (C′$_1$, C′$_2$), in order to deliver two video signals representing two images corresponding to the two viewpoints.

9. A method according to claim 8, when dependent on claim 4, characterized in that the two video signals are multiplexed for the purpose of their digitization and in that, for each image, the digitized positions are divided according to the camera from which the image concerned originated.

10. A method according to one of claims 1, 2, 3, 4, 5, 6, 7, 8 or 9, characterized in that the surface of the object is illuminated by sources formed by two separate laser devices ($16l_1$, $17l_2$).

11. A method according to one of claims 1 to 10, wherein the data representing the geometry of the video and light beam assembly consist of distances and angles ($p_1$, $p_2$, $c_1$, $c_2$) previously measured and stored in a memory (26), the specific data attached to each trace being the coordinates of the points of the said traces calculated by trigonometrical calculations.

12. A method according to claim 1, characterized in that the said calibration pattern consists of a plate (33a) carrying a grid of lines (33b) on one surface, the digitization being carried out for each laser plane by disposing the said plate (33a) parallel to the plane of the light beam in such a way that the lines intercept the said beam.

13. A method according to one of claims 1 or 12, characterized in that linear functions in the following form are calculated: $x = R(u, v) = \dfrac{au + bv + c}{gu + hv + 1}$ and $z = T(u, v) = \dfrac{du + ev + f}{gu + hv + 1}$, the coefficients (a-h) being determined by applying these equations to at least four points of the relief areas of the pattern, for which the quadruplet (x, z, u, v) is known and stored, and by solving the linear system of equations obtained.

14. A method according to one of claims 1 or 12, characterized in that non-linear functions in the following form are calculated:

$$x = R(u, v) = \frac{\displaystyle\sum_{ij=0}^{n} \alpha_{ij} u^i v^j}{1 + \displaystyle\sum_{ij=1}^{n} \gamma_{ij} u^i v^j}$$

$$z = T(u, v) = \frac{\displaystyle\sum_{ij=0}^{n} \beta_{ij} u^i v^j}{1 + \displaystyle\sum_{ij=1}^{n} \gamma_{ij} u^i v^j}$$

the coefficients $\alpha_{ij}$, $\beta_{ij}$, $\gamma_{ij}$ being determined by applying these equations to a number of points of the relief areas of the pattern, at least equal to $1 + \dfrac{3n(n+3)}{4}$, for which the quadruplet (x, z, u, v) is known and stored, and by solving the linear system of equations obtained.

15. A method according to one of claims 1 to 14, characterized in that the object is fixed and traversed by the axis of rotation (Z), and the video device and light beam assembly is caused to turn about the axis of rotation (Z).

16. Equipment for three-dimensional reading of the surface of an object (or subject) with the purpose of delivering a video signal representing the co-ordinates of the points of this surface, of the type comprising
   - a system of illumination of the object ($16l_1$, $17l_2$), adapted to form two sources ($L_1$, $L_2$) geometrically displaced along the axis of rotation, with the purpose of emitting towards the surface of the object two distinct flat beams extending in planes parallel to the axis of rotation (Z) or passing through this axis, the axes of the said beams forming two different angles ($l_1$, $l_2$) with the said axis of rotation (Z);
- a video device (11, $12c_1$, $13c_1$, $14c_1$, $15c_2$) mechanically integral with the illumination system, capable of filming the object with a parallax predetermined with respect to the beams of the illumination system, adapted to capture images of the object from two different viewpoints ($C_1$, $C_2$) geometrically displaced along the axis of rotation (Z) in such a way that the two corresponding filming angles ($c_1$, $c_2$) considered with respect to the said axis of rotation are different, so that they are capable of delivering one or more video signals corresponding to the said viewpoints;
- means (3, 10) of positioning the object in the trajectory of the beams of the illumination system and in the field of the video device;
- means (4-7) of displacing in relative rotation the illumination system and video device assembly with respect to the object abut an axis of rotation (Z);
the equipment being characterized in that the turning support (9) is provided with a centring mechanism (10) situated in the extension of the output shaft (7) and adapted to provide the positioning of the object; in that the centring mechanism (10) is adapted to receive removable tips of at least two types, one (10a, 34) being adapted to bear on the object to be digitized, and the other (36) being adapted to support and centre a calibration pattern (33) associated with the equipment; and in that it comprises means of calibration capable of digitizing the image of the surface of the said pattern and of calculating, from the results of this digitization, the functions x = R (u, v) and z = T (u, v), giving the metrical co-ordinates of the points of each laser plane as a function of the columns and lines of the corresponding image, the specific data attached to each trace being subsequently obtained by application of these functions.

17. Equipment according to claim 16 wherein, in combination,
- the video device comprises a video camera (11) specifically sensitive to a predetermined wavelength, and two sets of mirrors ($12c_1$, $13c_1$; $14c_1$, $15c_2$...) geometrically arranged to generate two viewpoints ($C_1$, $C_2$) situated in a median plane (M) containing the axis of rotation (Z), one of the sets providing a high filming angle and the other a low filming angle;
- the illumination system comprises two flat-beam laser devices ($16l_1$, $17l_1$) with a wavelength matched to that of the camera, arranged to form sources ($L_1$, $L_2$) disposed laterally on opposite sides of the aforesaid median plane (M), at different heights so that one illuminates at a high angle and the other at a low angle.

18. Equipment according to claim 17, characterized in that the sets of mirrors ($12c_1$, $13c_1$; $14c_2$, $15c_2$) are arranged to cover filming areas which are appreciably separated and correspond to half-spaces ($E_1$, $E_2$) respectively, situated on opposite sides of the median plane (M), the set of mirrors ($12c_1$, $13c_1$) providing the high filming angle ($c_1$) covering half-space ($E_1$) situated on the side of the laser source illuminating at a high angle ($L_1$), and conversely for the low angle.

19. Equipment according to one of claims 17 and 18, characterized in that it comprises a bracket (1), an electric motor (4) associated with a reduction gear unit (5) supported by the said bracket, a transmission system (6) associated with the said reduction gear unit and having an output shaft (7) collinear with the axis of rotation (Z), and a rotating support (9) carried by the output shaft, the video camera (11), the sets of mirrors ($12c_1$, $13c_1$; $14c_2$, $15c_2$) and the laser devices ($16l_1$, $17l_2$) being attached to the said rotating support (9).

20. Equipment according to one of claims 16 to 19, wherein an angular position detector (8) is associated with the means of rotary displacement (4-7), the said detector being adapted to deliver a signal representing the relative angular position of the illumination system and video device assembly with respect to the object.

21. Equipment according to claim 20, when dependent on claim 19, characterized in that the angular position detector (8) comprises a generator of electrical pulses with a frequency proportional to the rotation stored, coupled to the shaft of the electric motor.

22. Equipment according to claim 16, characterized in that the calibration pattern comprises a pattern (33a) having a grid of lines (33b).

23. Equipment according to one of claims 19 to 22, characterized in that the rotating support (9) has, on the one hand, a forward extension (9a) situated in front of the centring mechanism (10) and carrying the sets of mirrors ($12c_1$, $13c_1$; $14c_2$, $15c_2$) and the laser devices ($16l_1$, $17l_2$), and, on the other hand, an upper extension (9b) carrying the camera (11), the centring mechanism (10) being provided with an open section to free the optical path between the camera and the set of mirrors.

24. Equipment according to one of claims 16 to 23, adapted to make a reading of a human head or bust.

Fig. 1

Fig. 2

Fig. 3

Fig. 4

Fig. 5

Fig. 6

Fig.7

Fig. 8

Fig.9

Fig.10

$u_1 < u_m < u_2$

UT

Fig. 12

Fig. 11

Fig. 13

Fig. 14

Fig. 16

Fig.15a

10b

10

35

34

10a

Z

Fig. 15b

36

37

33b

33a

Z